(19) Europäisches Patentamt / European Patent Office / Office européen des brevets

(11) **EP 2 401 355 B1**

(12) **EUROPEAN PATENT SPECIFICATION**

(45) Date of publication and mention of the grant of the patent:
**05.09.2018 Bulletin 2018/36**

(51) Int Cl.:
*C12M 1/34* (2006.01)   *G01N 21/51* (2006.01)
*G01N 21/47* (2006.01)

(21) Application number: **10713503.0**

(22) Date of filing: **24.02.2010**

(86) International application number:
**PCT/IB2010/000373**

(87) International publication number:
**WO 2010/097687 (02.09.2010 Gazette 2010/35)**

(54) **METHOD FOR THE BACTERIOLOGICAL INVESTIGATION OF A BIOLOGICAL SAMPLE AND RELATIVE DEVICE**

VERFAHREN ZUR BAKTERIOLOGISCHEN UNTERSUCHUNG EINER BIOLOGISCHEN PROBE UND DAMIT VERBUNDENE VORRICHTUNG

PROCÉDÉ DE RECHERCHE DE BACTÉRIES DANS UN ÉCHANTILLON BIOLOGIQUE ET DISPOSITIF ASSOCIÉ

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **25.02.2009 IT UD20090048**

(43) Date of publication of application:
**04.01.2012 Bulletin 2012/01**

(73) Proprietor: **Alifax S.r.L.**
**35020 Polverara (PD) (IT)**

(72) Inventors:
• **GALIANO, Paolo**
**I-35100 Padova (IT)**
• **MANSUTTI, Alessandro**
**I-33013 Gemona del Friuli (IT)**

(74) Representative: **Petraz, Gilberto Luigi et al**
**GLP S.r.l.**
**Viale Europa Unita, 171**
**33100 Udine (IT)**

(56) References cited:
**WO-A-2006/021519     US-A1- 2005 254 055**

• **ROESSLER, WG. AND BREWER, C.R.: "Permanent Turbidity Standards" APPLIED MICROBIOLOGY, vol. 15, no. 5, September 1967 (1967-09), pages 1114-1121, XP002559667**

**Description**

FIELD OF THE INVENTION

The present invention concerns a method, and the relative device, for the bacteriological investigation of a biological sample intended to identify a bacterial load in the sample and to select the most effective antibiotic for the therapeutic treatment thereof. The sample to be analyzed can be, for example, urine, bronchial aspirate, blood, diluted blood or other.

BACKGROUND OF THE INVENTION

It is known that, according to current international guide lines, an in vitro test for sensitivity to antibiotics (antibiogram) provides to set up a standardized bacterial suspension to be tested against optimized concentrations (breakpoints) or scalar dilutions of antibiotics (MIC).

The number of bacteria analyzed must be standardized irrespective of the sensitivity of the method adopted for the test.

The preparation of an inoculum is one of the most critical passages of every sensitivity test or antibiogram. The inoculum can significantly influence the dimensions of the inhibition area.

According to the inoculum, falsely sensitive results can be obtained if too few bacteria are analyzed, whereas falsely resistant results can be obtained by analyzing too many bacteria.

For most microorganisms, the inoculum used should develop, after overnight incubation, semi-confluent colonies. An incorrect inoculum (colonies with a confluent or separate growth) can easily be recognized and these tests must therefore be repeated.

The preparation of an adequate inoculum is not critical until a semi-confluent growth is obtained.

Inoculums are generally prepared by adding to a cell culture medium from four to five isolated colonies of colonies with a similar morphology and afterwards by allowing them to grow in the logarithmic phase.

The selection of from four to five colonies, instead of from a single colony, is carried out to minimize the possibility of analyzing a colony that could be derived from a mutant-sensitive.

The inoculums can also be prepared directly by suspending colonies that have grown overnight on an agar dish, directly in culture medium or saline solution. This direct preparation technique of the inoculum with suspension is preferred for bacteria that grow in an unpredictable way in the medium, for example so-called fastidious bacteria.

Because growth in an inoculum medium is unreliable, it is obligatory to use fresh columns (from 16 to 24 hours).

The choice of the inoculation method is mainly conditioned by practical considerations, but the results are better if some standardization methods are adopted, such as the comparison of the density of the suspension of the microorganisms at a determinate standard of turbidity or an equivalent latex, or by carrying out photometric measurements.

In particular, the most widely used standardization method to standardize the inoculum provides McFarland turbidity standards, typically used in microbiology as a reference to regulate the turbidity of bacterial suspensions so as to have the number of bacteria within a certain range.

The McFarland standards (0.5, 1, 2, 3, 4) can be prepared by adding specific volumes of sulfuric acid or barium chloride dihydrate in order to obtain a solution of barium sulfate with a specific optical density.

The most commonly used standard is the 0.5 McFarland standard, which is prepared from 99.5 mL of sulfuric acid 1% added to 0.5mL of barium chloride dihydrate 1.175%, continuously stirred. This solution is dispensed inside test tubes similar to those used for the preparation of the inoculum, which are closed with screw-type stoppers and replaced in the dark at ambient temperature.

The 0.5 McFarland standard provides a standard that can be compared visually with that of a bacterial suspension, in sterile saline solution or growth medium, containing approximately $1.5*10^8$ CFU/ml.

The test tube to be analyzed with the inoculated medium or the direct suspension with the microorganisms is stirred.

Afterwards, by means of adequate illumination, the test tube is put adjacent to the 0.5 McFarland standard on a white background with black contrasting lines and their turbidities are compared, observing the black lines through the suspension.

If the suspension is too thick, it will be more difficult to observe the black lines than through the 0.5 McFarland standard. In this case, the inoculum is diluted with additional sterile medium or saline solution.

Otherwise, if the suspension to be tested is too "light", more microorganisms are added, or the suspension is incubated again (according to the inoculum preparation protocol) until the turbidity reaches the McFarland standard.

Once standardized, the suspension of the inoculum should be used within 15 minutes of being prepared.

Recently, suspensions of particles of latex have been used as simpler and more stable alternatives to barium sulfate, to obtain a turbidity comparable to that of the McFarland standard.

Alternatively, a standardization is made with a spectrophotometer or nephelometer, which is more expedient and more accurate than visual regulation for correspondence with the McFarland standard.

In this case the colonies are suspended in distilled water in a glass test tube so as to have a suspension with a visible turbidity.

The spectrophotometer is zeroed to 500 nm with sterile water or medium (also used for the suspension).

Afterward, the absorbance of the bacterial suspension is measured and, from a reference table, the volume to be transferred into 5 ml of sterile distilled water is selected and transferred using a suitable micropipette with a fixed volume.

This method depends on the type of spectrophotometer adopted, which can vary, just as it also depends on the choice of the type of test tubes or cuvettes of different sizes.

Therefore, to obtain a confluent growth, it is necessary to adapt, on each occasion, the dilutions to the instruments of each laboratory.

As we said, a nephelometer can also be used, but the instrument must be calibrated for the different groups of microorganisms.

Consequently, the known techniques of preparing the inoculum and comparing them with a 0.5 McFarland standard entail a considerable amount of work which is not without inaccuracies and slowness in obtaining the desired final data. From the above it is clear that, in the case of a patient whose treatment with antibiotics must be started quickly, the solutions known in the state of the art are not effective in supplying a precise datum quickly.

This leads to a lack of promptness with which, at times, an effective therapy is started in a patient, with the risk of even serious dangers to his health.

It is therefore common for physicians to administer in advance to the patient, without the support of diagnostic tests and exclusively according to a clinical suspicion, a broad-spectrum antibiotic to allow the therapy to be started immediately. The indiscriminate use of such antibiotics induces the so-called phenomenon of drug resistance.

The US patent application US-A-2005/0254055 (US'055) is known, which describes a method for monitoring, in real time and in line, the cell growth and the bacterial concentration, using the light scattering technique. Application US'055 is intended to control the growth of a microorganism that is already known from the start, and not to identify whether the bacterium is present or not and possibly what type it is. Application US'055, apart from being specifically intended for medium volume incubators or bio reactors, which is a very distant field from that of bacteriological analyses as in this case, also suffers from the disadvantage of the presence of air bubbles deriving from the stirring and mixing in the container of the sample to be analyzed: this makes it difficult to read the light scattering.

Purpose of the present invention is to perfect a method for bacteriological investigation and achieve a relative device, which allows to reduce the time needed to proceed with starting the antibiogram operation, so as to speed up the start of the therapeutic intervention on the patient, without determining any risk of causing drug resistance.

The Applicant has devised, tested and embodied the present invention to overcome the shortcomings of the state of the art and to obtain these and other purposes and advantages.

SUMMARY OF THE INVENTION

The present invention is set forth and characterized in the independent claims, while the dependent claims describe other characteristics of the invention or variants to the main inventive idea.

In accordance with the above purpose, a method for the bacteriological investigation of a biological sample according to the present invention provides to carry out a light-scattering reading in order to determine the turbidity according to the McFarland standard of a suspension of a liquid culture ground, or eugonic medium, into which the biological sample is inoculated and where the turbidity is measured continuously and directly by the suspension of the sample analyzed during the growth of the bacteria, until a determinate turbidity threshold is reached expressed according to the McFarland standard.

According to the present invention, the McFarland turbidity threshold is detected as having been reached by differential calculus from the start of the growth or incubation of the bacteria. Consequently, the present invention provides notice that the McFarland turbidity threshold has been reached in correspondence with a variation in turbidity, substantially given by the value calculated of the difference between the final and initial value of turbidity measured continuously during the replication phase. This has the advantage, for example compared with US'055, that it renders independent the reaching of the desired McFarland turbidity threshold from absolute turbidity.

According to a preferred form of embodiment, the method according to the invention comprises a first step in which the bacterial growth is determined in the suspension of the biological sample, inoculated in a liquid culture ground, contained in a containing element at least partly transparent to electromagnetic radiations.

Simultaneously with the bacterial growth, a coherent and collimated light beam (laser) is made to strike the containing element and the quantity of light refracted or diffused (light scattering) over time by the suspension is detected.

The detection is made in correspondence with a first and a second angular position, different from each other, with respect to the containing element, so as to determine a first and a second curve, respectively associated with the first and second angular position, of the development over time of the turbidity of the bacterial suspension.

The method according to the present invention also comprises a second step in which two differential curves are determined, given by the difference, respectively, between said first curve and the instantaneous value of turbidity at the start of the first step detected in correspondence with the first angular position, and between said second curve and the instantaneous value of turbidity at the start of the second step detected in correspondence with the second angular position.

The method then provides a third step in which, from the development of the two differential curves compared with first classification data, the type of bacterium present in the sample where bacterial growth occurs is deduced, or its family or the strain or species to which it belongs.

A fourth step is also provided in which the two differential curves are correlated to a corresponding variation in the turbidity values referred to the McFarland standard which defines said turbidity threshold, depending on pre-memorized second data regarding the dependence between the development of the two differential curves and the corresponding variation in the turbidity values referred to the McFarland standard. The second dependence data are defined and divided for each family or strain or species to which the bacteria belong.

One form of embodiment of the present invention adopts a particular reading unit based on the emission of coherent light or laser through a sample and reception of the relative diffused light in order to determine the presence and classification of bacteria.

Advantageously, the reading unit is able to signal, for example acoustically, or graphically or visually, when the McFarland turbidity threshold has been reached.

The method according to the present invention can be applied to detecting the McFarland turbidity on any glass or plastic container whatsoever, such as test tubes or micro-pits.

The present invention can be automated together with analyzers that use a laser light scattering reading system.

An advantageous feature of the present invention provides to control the correct measurement done by the reading unit by an automatic determination that the measurement of the McFarland turbidity has taken place, using suspensions of particles of latex with a known concentration.

With the present invention the time taken to start the antibiogram is reduced, so as to speed up the therapeutic intervention on the patient, without determining any risks of causing drug resistance.

In this way, the steps as described heretofore close with the availability of positive samples with a turbidity suitable to start the clinical antibiogram, that is, the tests for antibiotics directly from the growth medium.

This advantage allows to provide the clinician with the functional result of the first antibiotic tested (resistant or sensitive) in order to treat the patient correctly for the antibiotic administered if it were sensitive, or to change antibiotic if the test gave results showing resistance.

The invention therefore allows to perform an antibiogram of the clinical type, that is, an antibiogram performed directly on the growth medium inoculated with the biological sample examined which has proved positive to bacterial growth.

Furthermore, it is much more accurate to achieve the desired McFarland value, for example 0.5 standard, according to the invention than in the method using the dilution of the concentrated sample as performed in the state of the art. This is because it is more reliable to achieve a precise turbidity value starting from low values and operating during the bacterial growth.

The analysis time required by the present invention is considerably less than in state-of-the-art methods. The speed of detection is possible thanks to the measurement based on light scattering, which is much quicker and more sensitive in establishing in a short time the presence/absence of bacterial growths in the sample, thanks to the direct detection of turbidity and hence concentration of the organisms.

The present invention thus allows to provide precise and reliable results with a considerable saving in times compared with known methods, and can also be achieved with pre-existing machines and instruments.

In other words, the method allows to identify all the positives within a significantly reduced time compared with classic hemoculture methods.

This significantly reduces the average time of performing the antibiogram, with obvious benefits in the therapy and management of the patient.

The method according to the invention can be automated, requires a limited manual operativeness, thanks to the automation of the reading steps, data processing, display of results or other.

The present invention preferably uses vacuum-sealed sample containers and subjected to thermal autoclave, to prevent the growth of contaminants, unlike application US'055.

Furthermore, the present invention allows a dynamic measurement, avoiding the step of diluting the bacterial colonies and eliminating the disadvantage of the air bubbles generated during the mixing and stirring step which always affects application US'055, and which is not provided in the present invention.

A variant of the method according to the present invention provides to extrapolate, for time values of less than zero, a growth curve from the turbidity values measured continuously, and to detect that the McFarland turbidity threshold has been reached by differential calculus with respect to the minimum value of the curve extrapolated, so as to consider the contribution of turbidity of the microorganisms replicating before the start of the analysis as well.

BRIEF DESCRIPTION OF THE DRAWINGS

These and other characteristics of the present invention will become apparent from the following description of a preferential form of embodiment, given as a non-restrictive example with reference to the attached drawings wherein:

- fig. 1 is a schematic representation of a device according to the present invention;
- fig. 2 is a graph of the development over time (t) of the quantity of diffused light V1(t), V2(t) that is detected by two sensors S1, S2;
- fig. 3 is a graph of the development over time (t) of the variation $\Delta1(t)$, $\Delta2(t)$ of the quantity of diffused light V1(t), V2(t) that is detected by two sensors S1, S2 with respect to an initial value V1(0), V2(0);
- fig. 4 is a schematic representation of the method to detect the McFarland turbidity $\delta(t)$ starting from the curves $\Delta1(t)$, $\Delta2(t)$ in fig. 3.

DETAILED DESCRIPTION OF A PREFERENTIAL FORM OF EMBODIMENT

With reference to the attached drawings, a method according to the present invention is used to prepare, by means of laser light scattering technology, a biological sample in suspension in liquid culture medium, or eugonic medium, with a defined McFarland turbidity, in this case 0.5.

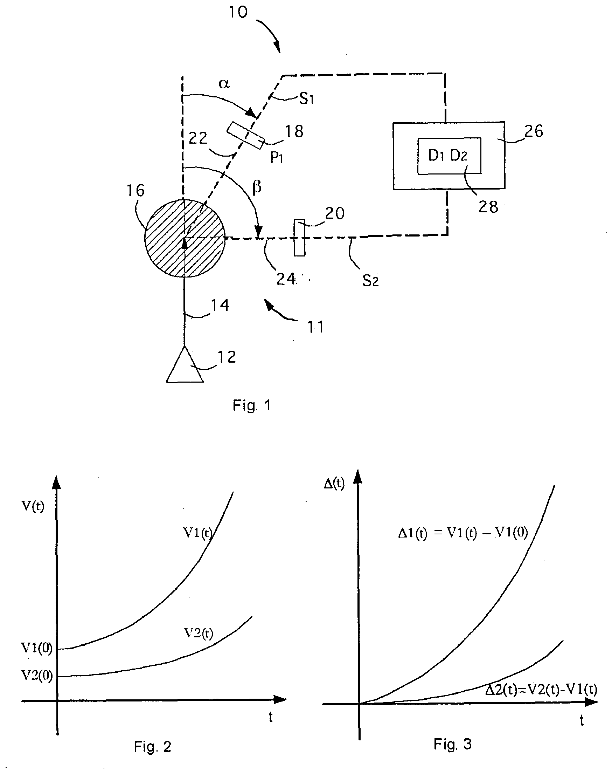

The method uses a device 10 (fig. 1) that provides to use a containing element or test tube 16, transparent to electromagnetic radiations, inside which the bacterial growth is provided of a suspension of the biological sample inoculated in the eugonic medium. The test tube 16 is advantageously provided closed, vacuum sealed and subjected to thermal autoclaving.

The device 10 comprises a reading unit 11 by means of which it detects the light diffused by the suspension of the sample contained in the test tube 16 and processing means 26, such as a computer, by means of which it processes the signals received by the reading unit 11 for the purposes of the analysis.

With the present invention, the sample is subjected to a culture test to verify it is positive to a determinate bacterial strain and, if positive, once prepared to the desired turbidity, is used directly in the subsequent steps of a clinical antibiogram test to assess in vitro sensitivity to antibiotics.

The measurement of the McFarland turbidity of the sample in the test tube by means of the reading unit 11 shown in fig. 1 is based on laser light emission and light scattering reading, directly in the analysis step, that is, at the same time as the bacterial growth during the culture test to determine the bacterial load in the sample analyzed.

In particular, the reading unit 11 is provided with emitter means 12 by means of which, on the test tube 16, a coherent or polarized (laser) and collimated beam 14 of light is made to strike.

Furthermore, the reading unit comprises sensor means, first 18 and second 20, by means of which rays 22, 24 of light diffused or refracted by the suspension are detected over time.

The first 18 and second 20 sensor means are able to generate corresponding signals S1, S2 which are transmitted to the processing means 26.

The first 18 and second 20 sensor means are located in correspondence with a first P1 and a second P2 angular position (fig. 1), different from each other, with respect to the test tube 16, so as to determine a first V1(t) and a second V2(t) curve, respectively associated with the first P1 and the second P2 angular position, of the development over time of the turbidity of the bacterial suspension.

In this case, the first 18 and second 20 sensor means are positioned at two predetermined angles a and b with respect to the direction of the beam 14, respectively at 30° and 90° (fig. 1).

The processing means 26 are able to process the signals S1, S2 produced by the first 18 and second 20 sensor means so as to determine, from the two curves V1(t) and V2(t), two differential curves $\Delta1(t)$ and $\Delta2(t)$ (fig. 3).

The two differential curves $\Delta1(t)$ and $\Delta2(t)$ are given by the difference respectively between the first curve V1(t) and a first instantaneous value V1(0) of turbidity at the start of the detection, detected in correspondence with the first position PI, and between the second curve V2(t) and a second instantaneous value V2(0) of turbidity at the start of the detection, detected in correspondence with the second position P2. The processing means 26 comprise memorization means 28 with a database in which first classification data D1 are memorized, by means of which, from the development of the two differential curves $\Delta1(t)$ and $\Delta2(t)$, the type of bacterium, or the family it belongs to, present in the sample where the bacterial growth takes place, is deduced.

The biological samples, with duplicating bacteria present, emit signals of diffused light that the reading unit 11 detects and the processing means 26 process so as to provide specific curves that express the development of the bacterial growth over time.

The first curve derived from the signal obtained from the first sensor 18 with an angle of 30° relates to the presence of bacteria and consequent measurement of the bacterial load over time.

The second curve derived from the signal obtained from the second sensor 20 with an angle of 90°, on the contrary, is more characterized by the morphology of the bacteria. From the growth curves V1(t), V2(t) of the possible bacterium obtained from the signals S1, S2 provided by the two sensors 18, 20, the two differential curves $\Delta1(t)$ and $\Delta2(t)$ are computed, which are given by the difference respectively between the first curve V1(t) and a first instantaneous value V1(0) of turbidity at the start of the detection, detected in correspondence with the first position PI, and between the second curve V2(t) and a second instantaneous value V2(0) of turbidity at the start of the detection, detected in correspondence with the second position P2.

From the differential curves $\Delta1(t)$ and $\Delta2(t)$ specific mathematical parameters are extrapolated for each curve, based on non-linear regression models. It is also possible to combine the homologous parameters of the two curves (for example by calculating ratios or differences or sums) in order to obtain further derived parameters.

The totality of these parameters (or sets) provides a synthetic description of the characteristics of the curve of the bacterium present in the sample analyzed.

An example of a regression equation applicable to the curves is one such as

$$\Delta(t) = a + b * e^{(c * t)}$$

which can be effectively used to describe the exponential growth development of a bacterial colony.

The equation can be applied to both the differential curves $\Delta1(t)$ and $\Delta2(t)$:

$$\Delta1(t) = a1 + b1 * e^{(c1 * t)}$$

$$\Delta2(t) = a2 + b2 * e^{(c2 * t)}$$

From these equations it is then possible to extrapolate the parameters a1, b1, c1, a2, b2 and c2. Afterward, the processing means 26 compare the first data D1 with the parameters extrapolated from the differential curves $\Delta1(t)$ and $\Delta2(t)$. In this case the first data D1 consist of the collection of the typical values of said sets of parameters of the regression equations applied to the growth curves (including the validity ranges thereof) for various types of bacteria, or for bacteria of the coccus or bacillus type, or for different families of bacteria, or for different bacterial species or for different bacterial strains.

In particular, the processing means 26 compare the set of parameters of the bacterium studied with the various sets of parameters present in the data D1 determining, with the aid of suitable statistical techniques, which type or family or species or strain is, with the greatest probability, the one to which the bacterium in question belongs.

In the memorization means 26, second data D2 are stored, regarding the dependence between the development of the two differential curves $\Delta1(t)$ and $\Delta2(t)$ and turbidity values according to the McFarland standard.

The second dependence data D2 are defined and divided for each family to which the bacteria belong. The data D2 are the parameters of the dependence relationships, for the various families, between the increase in turbidity measured with the scattering technique and the relative increase in McFarland turbidity.

The processing means 26 are able to correlate the two differential curves $\Delta1(t)$ and $\Delta2(t)$ to a defined value of turbidity according to the McFarland standard, advantageously McFarland 0.5.

The reading value detected by the reading unit 11 at the beginning of the analysis (t=0) is memorized and a reference McFarland turbidity level is conventionally assigned to it, in this case zero (0). Experimentally a relationship of dependence was determined between the increase in the value detected by the reading unit 11 (expressed as difference or delta $\Delta$ with respect to the value memorized initially) and the relative variation in turbidity expressed in McFarland units.

The values detected by the two sensors 18, 20 at the start of the analysis, that is, at time zero (t=0), are indicated conventionally as V1(0) and V2(0) and are sent to the processing means 26 where they are memorized in the memorization means 28.

In the course of the analysis, the first 18 and second 20 sensor means detect the quantity of light refracted on the two angles a and b in which they are with respect to the test tube 16. Given that t is a generic instant of time during the analysis, the values read by the first 18 and second 20 sensor means at time t are conventionally indicated as V1(t) and V2(t), indicating by this the development or function of the light diffused with respect to time t.

Fig. 2 shows the typical exponential temporal development of the curve associated with the functions V1(t) and V2(t) of the values detected by the first 18 and second 20 sensor means during the analysis of a biological sample containing bacteria growing in a eugonic medium.

According to the present invention, at time t the difference is calculated for each sensor, between the current value V(t) and the value at instant t=0, that is, for the first sensor 18 we have the formula:

$$\Delta 1(t) = V1(t) - V1(0)$$

whereas for the second sensor 20 we have:

$$\Delta 2(t) = V2(t) - V2(0)$$

It is therefore possible to obtain the two curves $\Delta 1(t)$ and $\Delta 2(t)$ (shown in fig. 3) which represent the variation in scattering detected by the two sensors over time.

Applicant has identified a relationship of dependence between the variation in turbidity measured with the light-scattering technology by means of the reading unit 11 (indicated by the symbol $\Delta$) and the same variation in turbidity expressed in McFarland units (indicated by $\delta$, whereas the relative development in time, or function of time, is indicated by $\delta(t)$). This dependence is not fixed, but depends on various factors, including the form and size of the bacterium in question.

Applicant has examined experimentally the growth of different families of bacteria, detected as described with reference to fig. 1. In particular, the developments of the variation in scattering $\Delta 1(t)$ and $\Delta 2(t)$ were examined, comparing them with the increase in the turbidity value expressed in McFarland ($\delta$) detected by means of a reference photometer.

From these tests the dependences were extrapolated for each bacterial family examined, between the increase in the scattering value detected on the two sensors 18, 20 $\Delta 1(t)$ and $\Delta 2(t)$, and the relative increase in turbidity expressed in McFarland units ($\delta$). The information on the dependences, second data D2, was memorized in the memorization means 28.

To give an example, the second data D2 can have a structure as shown in the following Table:

<div align="center">Table</div>

| Family of the bacterium | Dependence relationship |
|---|---|
| Family 1 | $\delta = a1* \Delta^2 + b1* \Delta + c1$ |
| Family 2 | $\delta = a2* \Delta^2 + b2* \Delta + c2$ |
| Family 3 | $\delta = a3* \Delta^2 + b3* \Delta + c3$ |

In the Table, with every family of bacteria a dependence relationship is associated, which links the variation in turbidity expressed in McFarland units ($\delta$) and the relative variation in turbidity measured according to the light-scattering technique ($\Delta$).

Therefore, if we know the family to which the bacterium studied belongs (e.g. Family 1), and if we know the variation in turbidity as measured according to the light-scattering technique starting from the initial instant t=0 ($\Delta$), the corresponding variation in turbidity expressed in McFarland units ($\delta$) is:

$$\delta = a1* \Delta^2 + b1* \Delta + c1$$

where a1, b1 and c1 are suitable specific parameters of the family identified.

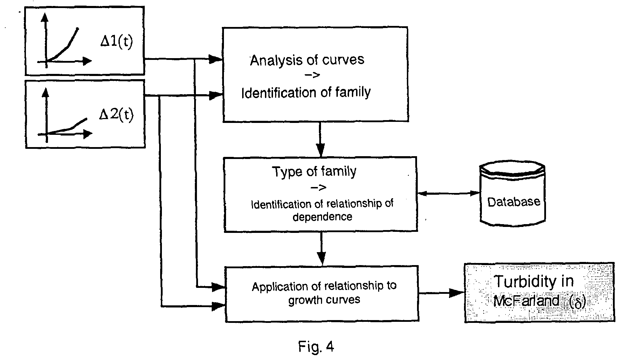

Fig. 4 shows schematically the detection principle of McFarland turbidity $\delta(t)$ starting from the curves $\Delta 1(t)$ and $\Delta 2(t)$. From the comparative analysis, based on the first data D1, of the curves $\Delta 1(t)$ and $\Delta 2(t)$ it is possible to trace the type of bacterium examined or at least the family it belongs to.

According to the family it belongs to it is possible to define the dependence relationship between $\Delta$ and $\delta$, starting from the database of the laboratory experiments.

At this point, since we know the dependence relationship, it is possible to calculate the current McFarland turbidity value $\delta$ of the sample analyzed, applying said formula to the two curves $\Delta 1(t)$ and $\Delta 2(t)$

Preferably, latexes with a known concentration are also provided, as a further confirmation of the measurement done.

The practical application of this technique allows to detect whether a determinate McFarland turbidity level has been reached (in this case 0.5 units), which is necessary as a standardized concentration for the inoculum of the antibiogram test during the growth of the positive sample subjected to culture analysis.

The continuous monitoring of the turbidity of the sample during the growth gives operating advantages which allow to speed up the inoculation procedures for the clinical or standard antibiogram test.

This because the standard procedures require, as is known, that colonies previously isolated in a dish must be diluted in physiological/saline solutions until suspensions with a standardized turbidity are obtained. Therefore, the known methodology can result in errors of dilution, handling or choice of colonies used. These preparations are normally obtained when said bacteria have already grown in the normal Petri dishes that require at least an overnight incubation.

With the new technique as described here, therefore, all those manual operations relating to the preparation of the bacterial suspension are avoided, thus reducing the possible factors of error and enabling a greater automation of the test.

The time taken to reach a predetermined McFarland turbidity level, for example 0.5, of the sample during the growth step depends on the initial bacterial load of the sample, where the greater the count value, the quicker the preestablished McFarland threshold will be reached.

Advantageously, the reading unit 11 and the relative device 10 for preparing the sample to the desired level of turbidity as described can be integrated in a single automatic instrument.

According to an advantageous variant of the present invention the method is applied, as well as in the case of bacterial growth with a lag phase with a flat curve starting at time t=0, where the lowest value of the curve is equal to the initial value V(0) at time t=0, also when the bacteria are already at the replication phase (absence of lag-phase) before the analysis.

In this case, the growth curve extrapolated starting from the growth values read is qualitatively shifted backward, that is, to the left with respect to the direction of the temporal x-axis, with respect to the curve with lag-phase and has a turbidity value V(0) at the time the analysis is started t=0 which is not the minimum starting value of the curve, as, on the contrary, in the case of the flat curve starting with lag-phase.

In this situation, the present invention can calculate the difference in turbidity no longer with respect to the value V(0) at initial time (t=0), but with respect to the actual lowest value Vmin of the curve extrapolated starting from the bacterial growth values read continuously, that is, extrapolated for temporal values of less than zero t<0. This choice is advantageous because it also takes into account the contribution, in terms of turbidity, given by the bacteria that had already begun to replicate, thus giving their turbidity contribution, before the analysis had started, that is, before time t=0.

## Claims

1. Method for the bacteriological investigation of a biological sample intended to identify a bacterial load in the sample and to select the most effective antibiotic for the therapeutic treatment thereof, **characterized in that** it provides to carry out a light scattering reading in order to determine the turbidity according to the McFarland standard of a suspension formed from a liquid culture means or eugonic medium in which the biological sample is inoculated, and in which the turbidity is continuously measured directly from the suspension of the sample analyzed during the growth step of the bacteria, until a determined threshold of turbidity is reached expressed according to the McFarland standard, and **in that** it detects that the McFarland turbidity threshold has been reached through differential calculus from the start of the growth step of the bacteria, providing notice that the McFarland turbidity threshold has been reached in correspondence with a variation in turbidity, substantially given by the value calculated of the difference between the final and initial value of turbidity measured continuously during the replication phase, independently from the reaching of the desired McFarland turbidity threshold from absolute turbidity.

2. Method as in claim 1, comprising:

   - a first step in which the bacterial growth is carried out in the suspension of the biological sample inoculated in the liquid culture means contained in a containing element (16) at least partly transparent to electromagnetic radiations, on which containing element (16), simultaneously to the bacterial growth, a coherent and collimated beam (14) of light is made to strike, and the amount of light refracted or diffused by the suspension is detected over time, said detection being carried out in correspondence with a first (P1) and a second (P2) angular position, different from each other, with respect to said containing element (16), so as to determine a first V1(t) and a second V2(t) curve, respectively associated with the first (P1) and the second (P2) angular position, of the development over time of the turbidity of the bacterial suspension;

   **characterized in that** it comprises:

- a second step in which two differential curves Δ1(t) and Δ2(t) are determined, given by the difference respectively between said first curve V1(t) and a first instantaneous value V1(0) of turbidity at the beginning of the first step, detected in correspondence with the first (P1) angular position and between said second curve V2(t) and a second instantaneous value V2(0) of turbidity at the beginning of the first step, detected in correspondence with the second (P2) angular position;

- a third step in which, from the development of the two differential curves Δ1(t) and Δ2(t) compared with first classification data (D1) consisting of the collection of the typical values of sets of parameters of the regression equations applied to the growth curves for various types of bacteria, or for bacteria of the coccus or bacillus type, or for different families of bacteria, or for different bacterial species or for different bacterial strains, the type of bacterium present in the sample or its family or strain or species to which it belongs is deduced;

- a fourth step in which the two differential curves Δ1(t) and Δ2(t) are correlated to a corresponding variation δ of the turbidity values referred to the McFarland standard δ(t) which defines said turbidity threshold, according to pre-memorized second data (D2) of dependence between the development of the two differential curves Δ1(t) and Δ2(t) and turbidity values according to the McFarland standard δ(t), said second data (D2) being defined and divided for each family or strain or species to which the bacteria belong, wherein said dependence is expressed by the relationship $\delta = a1^* \Delta^2 + b1^* \Delta + c1$ where a1, b1 and c1 are suitable specific parameters of the family identified.

3. Method as in claim 1, or 2, **characterized in that** the variation δ of the fourth step corresponds to the 0.5 McFarland standard.

4. Method as in any claim hereinbefore, **characterized in that** it provides a step to control the correct measurement of McFarland turbidity by means of a comparison with suspensions of particles of latex with a known concentration.

5. Method as in any claim hereinbefore, **characterized in that** it provides to extrapolate, for temporal values of less than zero, a growth curve from the turbidity values measured continuously, and to detect that the McFarland turbidity threshold has been reached by a differential calculus with respect to the minimum value of the curve extrapolated, so as to consider also the contribution of turbidity of the microorganisms replicating before the start of the analysis.

6. Device for the bacterial investigation of a biological sample intended to identify a bacterial load in the sample and to select the most effective antibiotic for the therapeutic treatment thereof, **characterized in that** it comprises:

- a containing element (16) at least partly transparent to electromagnetic radiations, inside which the bacterial growth of a suspension of the biological sample inoculated in a liquid culture means or eugonic medium is provided;

- a reading unit (11) provided with emitter means (12) by means of which, on said containing element (16), a coherent and collimated beam of light (14) is made to strike, and with first (18) and second (20) sensor means by means of which beams (22, 23) of light refracted or diffused by the suspension are detected over time, said first (18) and second (20) sensor means being located in correspondence with a first (P1) and a second (P2) angular position, different from each other, with respect to said containing element (16), so as to determine a first V1(t) and a second V2(t) curve, respectively associated with the first (P1) and the second (P2) angular position, of the development over time of the turbidity of the bacterial suspension, said turbidity being continuously measured during the replication phase;

- processing means (26) able to process signals produced by the first (18) and second (20) sensor means so as to determine two differential curves Δ1(t) and Δ2(t) given by the difference respectively between said first curve V1(t) and a first instantaneous value V1(0) of turbidity at the beginning of the detection, detected in correspondence with the first (P1) angular position and between said second curve V2(t) and a second value V2(0) of instantaneous turbidity at the beginning of the detection, detected in correspondence with the second angular position (P2), said processing means (26) comprising data base memorization means (28) in which first classification data (D1) consisting of the collection of the typical values of sets of parameters of the regression equations applied to the growth curves for various types of bacteria, or for bacteria of the coccus or bacillus type, or for different families of bacteria, or for different bacterial species or for different bacterial strains are memorized by means of which, from the development of the two differential curves Δ1(t) and Δ2(t) the type of bacterium present in the biological sample or its family, or strain or species to which it belongs is deduced, there also being in said memorization means (28) pre-memorized second data (D2) of dependence between the development of the two differential curves Δ1(t) and Δ2(t) and a corresponding variation δ of the turbidity values according to the McFarland standard δ(t) which defines a desired turbidity threshold that is obtained independently from the reaching of the desired McFarland turbidity threshold from absolute turbidity, said second dependence data (D2) being defined and divided for each family, or strain or species to which the bacteria belong, wherein said dependence is expressed by the relationship $\delta = a1^* \Delta^2 + b1^* \Delta + c1$ where a1, b1 and c1 are suitable specific parameters of the family identified, said processing means (26) being able to correlate the two differential curves $\Delta 1(t)$ and $\Delta 2(t)$ to the corresponding variation $\delta$ of said turbidity values according to the McFarland standard.

**Patentansprüche**

1. Verfahren zur bakteriologischen Untersuchung einer biologischen Probe, welches dazu vorgesehen ist, eine bakterielle Last in der Probe zu ermitteln und das effektivste Antibiotikum für deren therapeutische Behandlung auszuwählen,
wobei das Verfahren **dadurch gekennzeichnet ist,**
**dass** es vorsieht, eine Lichtstreuungsmessung durchzuführen, um die Trübung einer Suspension nach dem McFarland-Standard zu bestimmen, wobei die Suspension aus einem flüssigen Kulturmittel oder eugonischen Medium gebildet ist, in welchem die biologische Probe inokuliert wird und in welchem die Trübung kontinuierlich direkt in der Suspension der Probe gemessen wird, die während des Wachstumsschritts der Bakterien analysiert wird, bis eine bestimmte Trübungsschwelle nach dem McFarland-Standard erreicht ist, und
**dass** es durch differenzielle Rechnung vom Beginn des Wachstumsschrittes der Bakterien an ermittelt, dass die McFarland-Trübungsschwelle erreicht wurde,
wobei anhand einer Änderung der Trübung und unabhängig von dem Erreichen der erwünschten McFarland-Trübungsschwelle anhand der absoluten Trübung festgestellt wird, dass die McFarland-Trübungsschwelle erreicht wurde, wobei die Änderung der Trübung im Wesentlichen durch den Wert bestimmt ist, welcher aus der Differenz zwischen dem Endwert und dem Anfangswert der Trübung berechnet wird, welche kontinuierlich während der Replikationsphase gemessen wird.

2. Verfahren nach Anspruch 1, welches umfasst:

- einen ersten Schritt, in welchem das bakterielle Wachstum in der Suspension der biologischen Probe durchgeführt wird, welche in dem flüssigen Kulturmittel inokuliert wird, welches in einem Behälterelement (16) enthalten ist, welches zumindest teilweise transparent gegenüber elektromagnetischer Strahlung ist, wobei auf das Behälterelement (16), parallel zu dem Bakterienwachstum, ein kohärenter und kollimierter Lichtstrahl (14) zum Auftreffen gebracht wird, und die Menge von durch die Suspension gebrochenem oder gestreutem Licht über die Zeit erfasst wird, wobei die Erfassung entsprechend einer ersten (P1) und einer zweiten (P2) Winkelposition bezogen auf das Behälterelement (16), welche unterschiedlich sind, durchgeführt wird, um eine erste V1(t) und eine zweite V2(t) Kurve des zeitlichen Verlaufs der Trübung der bakteriellen Suspension, welche jeweils der ersten (P1) und der zweiten (P2) Winkelposition zugeordnet sind, zu bestimmen;

wobei das Verfahren **dadurch gekennzeichnet ist, dass** es umfasst:

- einen zweiten Schritt, in dem zwei differenzielle Kurven $\Delta 1(t)$ und $\Delta 2(t)$ bestimmt werden, welche jeweils durch die Differenz zwischen der ersten Kurve V1(t) und einem ersten instantanen Wert V1(o) der Trübung zu Beginn des ersten Schrittes, welche entsprechend der ersten (P1) Winkelposition erfasst werden, und durch die Differenz zwischen der zweiten Kurve V2(t) und einem zweiten instantanen Wert V2(o) der Trübung zu Beginn des ersten Schrittes, welche entsprechend der zweiten (P2) Winkelposition erfasst werden, gegeben sind;
- einen dritten Schritt, in welchem aus dem Verlauf der zwei differenziellen Kurven $\Delta 1(t)$ und $\Delta 2(t)$ im Vergleich zu ersten Klassifizierungsdaten (D1), welche aus der Sammlung von den typischen Werten von Sätzen von Parametern der Regressionsgleichungen bestehen, welche auf die Wachstumskurven für unterschiedliche Arten von Bakterien oder für Bakterien von der Kokken- oder Bazillenart oder für unterschiedliche Familien von Bakterien oder für unterschiedliche bakterielle Gattungen oder für unterschiedliche bakterielle Stämme angewendet werden, die Art des Bakteriums, welches in der Probe vorhanden ist, oder seine Familie oder sein Stamm oder seine Gattung, zu welcher es gehört, ermittelt wird;
- einen vierten Schritt, in welchem die zwei differenziellen Kurven $\Delta 1(t)$ und $\Delta 2(t)$ mit einer zugehörigen Änderung $\delta$ der Trübungswerte bezogen auf den McFarland-Standard $\delta(t)$, welcher den Trübungsschwellenwert definiert, gemäß vorher gespeicherten zweiten Daten (D2) einer Abhängigkeit zwischen dem Verlauf der zwei differenziellen Kurven $\Delta 1(t)$ und $\Delta 2(t)$ und Trübungswerten nach dem McFarland-Standard $\delta(t)$ korreliert werden, wobei die zweiten Daten (D2) für jede Familie oder jeden Stamm oder jede Gattung, zu der die Bakterien gehören, definiert und aufgeteilt sind, wobei die Abhängigkeit durch den Zusammenhang $\delta = a1^* \Delta^2 + b1^* \Delta + c1$ ausge-

drückt wird, wobei a1, b1 und c1 passende spezifische Parameter der identifizierten Familie sind.

3. Verfahren nach Anspruch 1 oder 2, welches **dadurch gekennzeichnet ist, dass** die Änderung δ des vierten Schritts dem 0,5-McFarland-Standard entspricht.

4. Verfahren nach einem der vorhergegangenen Ansprüche, welches **dadurch gekennzeichnet ist, dass** es einen Schritt vorsieht, um die korrekte Messung einer McFarland-Trübung mithilfe eines Vergleichs mit Suspensionen von Latexpartikeln mit einer bekannten Konzentration zu steuern.

5. Verfahren nach einem der vorhergegangenen Ansprüche, welches **dadurch gekennzeichnet ist, dass** es vorsieht, eine Wachstumskurve aus den kontinuierlich gemessenen Trübungswerten für Zeitwerte kleiner als 0 zu extrapolieren und durch eine differenzielle Rechnung bezogen auf den Minimalwert der extrapolierten Kurve zu erkennen, dass die McFarland-Trübungsschwelle erreicht wurde, um auch den Beitrag von Mikroorganismen, welche sich vor dem Start der Analyse vermehren, zu der Trübung mit einzubeziehen.

6. Vorrichtung zur bakteriellen Untersuchung einer biologischen Probe, welche dafür vorgesehen ist, eine bakterielle Last in der Probe zu ermitteln und das effektivste Antibiotikum für deren therapeutische Behandlung auszuwählen, wobei die Vorrichtung **dadurch gekennzeichnet ist, dass** sie umfasst:

- ein Behälterelement (16), welches zumindest teilweise transparent gegenüber elektromagnetischer Strahlung ist und in welchem das bakterielle Wachstum einer Suspension der biologischen Probe, welche in einem flüssigen Kulturmedium oder in einem eugonischen Medium inokuliert wird, vorgesehen ist;
- eine Messeinheit (11), welche mit Emittermitteln (12), mit welchen ein kohärenter und kollimierter Lichtstrahl (14) zum Auftreffen auf das Behälterelement (16) gebracht wird, und mit ersten (18) und zweiten (20) Sensormitteln versehen ist, mithilfe derer Strahlen (22, 23) von durch die Suspension gebrochenem oder gestreutem Licht zeitabhängig erfasst werden, wobei die ersten (18) und zweiten (20) Sensormittel entsprechend einer ersten (P1) und einer zweiten (P2) Winkelposition bezogen auf das Behälterelement (16), welche unterschiedlich sind, angeordnet sind, um eine erste V1(t) und eine zweite V2(t) Kurve des zeitlichen Verlaufs der Trübung der bakteriellen Suspension zu bestimmen, welche jeweils der ersten (P1) und der zweiten (P2) Winkelposition zugeordnet sind, wobei die Trübung während der Replikationsphase kontinuierlich gemessen wird;
- Verarbeitungsmittel (26) welche dazu eingerichtet sind, Signale, welche von den ersten (18) und zweiten (20) Sensormitteln erzeugt werden, zu verarbeiten, um zwei differenzielle Kurven Δ1(t) und Δ2(t) zu bestimmen, welche jeweils durch die Differenz zwischen der ersten Kurve V1(t) und einem ersten instantanen Wert V1(o) der Trübung zu Beginn der Erfassung, welche entsprechend der ersten (P1) Winkelposition erfasst werden, und durch die Differenz zwischen der zweiten Kurve V2(t) und einem zweiten Wert V2(o) der instantanen Trübung zu Beginn der Erfassung, welche entsprechend der zweiten (P2) Winkelposition erfasst werden, gegeben sind,

wobei die Verarbeitungsmittel (26) Datenbank-Speichermittel (28) umfassen, in welchen erste Klassifizierungsdaten (D1) gespeichert werden, welche aus der Sammlung von den typischen Werten von Sätzen von Parametern von den Regressionsgleichungen bestehen, die auf die Wachstumskurven für unterschiedliche Arten von Bakterien oder für Bakterien der Kokken- oder Bazillen-Art oder für unterschiedliche Familien von Bakterien oder für unterschiedliche bakterielle Gattungen oder für unterschiedliche bakterielle Stämme angewandt werden, mithilfe deren aus dem Verlauf der zwei differenziellen Kurven Δ1(t) und Δ2(t) die Art des Bakteriums, welches in der biologischen Probe vorhanden ist, oder seine Familie oder sein Stamm oder seine Gattung, zu welcher es gehört, ermittelt wird, wobei in den Speichermitteln (28) auch vorher gespeicherte zweite Daten (D2) einer Abhängigkeit zwischen dem Verlauf der zwei differenziellen Kurven Δ1(t) und Δ2(t) und einer entsprechenden Änderung δ der Trübungswerte nach dem McFarland-Standard δ(t) vorhanden sind, welcher einen gewünschten Trübungsschwellwert definiert, der unabhängig von dem Erreichen des gewünschten McFarland-Trübungsschwellwerts anhand der absoluten Trübung erhalten wird, wobei die zweiten Abhängigkeitsdaten (D2) für jede Familie oder jeden Stamm oder jede Gattung, zu welcher die Bakterien gehören, definiert und eingeteilt sind, wobei die Abhängigkeit durch den Zusammenhang $\delta = a1 * \Delta^2 + b1 * \Delta + c1$ ausgedrückt wird, wobei a1, b1 und c1 passende spezifische Parameter der identifizierten Familie sind, wobei die Verarbeitungsmittel (26) dazu eingerichtet sind, die zwei differenziellen Kurven Δ1(t) und Δ2(t) mit der entsprechenden Änderung δ der Trübungswerte nach dem McFarland-Standard zu korrelieren.

**Revendications**

1. Procédé de recherche de bactéries dans un échantillon biologique destiné à identifier une charge bactérienne dans l'échantillon et à sélectionner l'antibiotique le plus efficace pour le traitement thérapeutique de celle-ci, **caractérisé par le fait qu'**il prévoit de réaliser une mesure de diffusion de lumière afin de déterminer la turbidité selon le standard McFarland d'une suspension formée à partir d'un moyen de culture liquide ou d'un milieu eugonique dans lequel l'échantillon biologique est inoculé, et dans lequel la turbidité est mesurée de manière continue directement à partir de la suspension de l'échantillon analysé pendant l'étape de croissance des bactéries, jusqu'à ce qu'un seuil de turbidité déterminé soit atteint, exprimé selon le standard McFarland, et **par le fait qu'**il détecte que le seuil de turbidité McFarland a été atteint par calcul différentiel depuis le début de l'étape de croissance des bactéries, indiquant que le seuil de turbidité McFarland a été atteint en correspondance avec une variation dans la turbidité, donnée sensiblement par la valeur calculée de la différence entre la valeur finale et la valeur initiale de la turbidité mesurée de manière continue pendant la phase de réplication, indépendamment du fait que le seuil de turbidité de McFarland souhaité soit atteint à partir de la turbidité absolue.

2. Procédé selon la revendication 1, comprenant :

   - une première étape dans laquelle la croissance bactérienne est conduite dans la suspension de l'échantillon biologique inoculé dans le moyen de culture liquide contenu dans un élément de réception (16) au moins partiellement transparent aux rayonnements électromagnétiques, un faisceau cohérent et collimaté (14) de lumière étant amené à être incident sur l'élément de réception (16) simultanément à la croissance bactérienne, et la quantité de lumière réfractée ou diffusée par la suspension est détectée au cours du temps, ladite détection étant réalisée en correspondance avec une première position angulaire (P1) et une seconde position angulaire (P2), différentes l'une de l'autre, par rapport audit élément de réception (16), de façon à déterminer une première courbe V1(t) et une seconde courbe V2(t), associées respectivement à la première position angulaire (P1) et à la seconde position angulaire (P2), du développement, au cours du temps, de la turbidité de la suspension bactérienne ;

   **caractérisé par le fait qu'**il comprend :

   - une deuxième étape dans laquelle deux courbes différentielles $\Delta1(t)$ et $\Delta2(t)$ sont déterminées, données par la différence, respectivement, entre ladite première courbe V1(t) et une première valeur instantanée V1(0) de turbidité au début de la première étape, détectée en correspondance avec la première position angulaire (P1), et entre ladite seconde courbe V2(t) et une seconde valeur instantanée V2(0) de turbidité au début de la première étape, détectée en correspondance avec la seconde position angulaire (P2) ;
   - une troisième étape dans laquelle, à partir du développement des deux courbes différentielles $\Delta1(t)$ et $\Delta2(t)$ en comparaison avec des premières données de classification (D1) consistant en la collection des valeurs typiques d'ensembles de paramètres des équations de régression appliquées aux courbes de croissance pour divers types de bactéries, ou pour des bactéries du type cocci ou bacille, ou pour différentes familles de bactéries, ou pour différentes espèces bactériennes ou pour différentes souches bactériennes, le type de bactérie présent dans l'échantillon ou sa famille ou souche ou espèce à laquelle elle appartient est déduit ;
   - une quatrième étape dans laquelle les deux courbes différentielles $\Delta1(t)$ et $\Delta2(t)$ sont mises en corrélation avec une variation correspondante $\delta$ des valeurs de turbidité en référence au standard McFarland 5(t) qui définit ledit seuil de turbidité, selon des secondes données pré-mémorisées (D2) de dépendance entre le développement des deux courbes différentielles $\Delta1(t)$ et $\Delta2(t)$ et des valeurs de turbidité selon le standard McFarland $\delta(t)$, lesdites secondes données (D2) étant définies et divisées pour chaque famille ou souche ou espèce à laquelle les bactéries appartiennent, ladite dépendance étant exprimée par la relation $\delta = a1 * \Delta^2 + b1 * \Delta + c1$, où a1, b1 et c1 sont des paramètres spécifiques appropriés de la famille identifiée.

3. Procédé selon la revendication 1 ou 2, **caractérisé par le fait que** la variation $\delta$ de la quatrième étape correspond au standard McFarland 0,5.

4. Procédé selon l'une quelconque des revendications précédentes, **caractérisé par le fait qu'**il comprend une étape pour contrôler la mesure correcte de la turbidité McFarland au moyen d'une comparaison avec des suspensions de particules de latex ayant une concentration connue.

5. Procédé selon l'une quelconque des revendications précédentes, **caractérisé par le fait qu'**il prévoit d'extrapoler, pour des valeurs temporelles inférieures à zéro, une courbe de croissance à partir des valeurs de turbidité mesurées de manière continue, et de détecter que le seuil de turbidité McFarland a été atteint par un calcul différentiel par rapport à la valeur minimale de la courbe extrapolée, de façon à tenir également compte de la contribution à la turbidité des micro-organismes en réplication avant le début de l'analyse.

6. Dispositif de recherche bactérienne dans un échantillon biologique destiné à identifier une charge bactérienne dans l'échantillon et à sélectionner l'antibiotique le plus efficace pour le traitement thérapeutique de celle-ci, **caractérisé par le fait qu'**il comprend :

- un élément de réception (16) au moins partiellement transparent aux rayonnements électromagnétiques, à l'intérieur duquel la croissance bactérienne d'une suspension de l'échantillon biologique inoculé dans un moyen de culture liquide ou dans un milieu eugonique est réalisée ;
- une unité de mesure (11) munie de moyens d'émetteur (12), au moyen desquels un faisceau cohérent et collimaté de lumière (14) est amené à être incident sur ledit élément de réception (16), et de premiers moyens de capteur (18) et de seconds moyens de capteur (20) au moyen desquels des faisceaux (22, 23) de lumière réfractée ou diffusée par la suspension sont détectés au cours du temps, lesdits premiers moyens de capteur (18) et lesdits seconds moyens de capteur (20) étant situés en correspondance avec une première position angulaire (P1) et une seconde position angulaire (P2), différentes l'une de l'autre, par rapport audit élément de réception (16), de façon à déterminer une première courbe V1(t) et une seconde courbe V2(t), associées respectivement à la première position angulaire (P1) et à la seconde position angulaire (P2), du développement, au cours du temps, de la turbidité de la suspension bactérienne, ladite turbidité étant mesurée de manière continue pendant la phase de réplication ;
- des moyens de traitement (26) aptes à traiter des signaux produits par les premiers moyens de capteur (18) et les seconds moyens de capteur (20) de façon à déterminer deux courbes différentielle $\Delta1(t)$ et $\Delta2(t)$ données par la différence, respectivement, entre ladite première courbe V1(t) et une première valeur instantanée V1(0) de turbidité au début de la détection, détectée en correspondance avec la première position angulaire (P1), et entre ladite seconde courbe V2(t) et une seconde valeur V2(0) de turbidité instantanée au début de la détection, détectée en correspondance avec la seconde position angulaire (P2), lesdits moyens de traitement (26) comprenant des moyens de mémorisation de base de données (28) dans lesquels des premières données de classification (D1) consistant en la collection des valeurs typiques d'ensembles de paramètres des équations de régression appliquées aux courbes de croissance pour divers types de bactéries, ou pour des bactéries du type cocci ou bacille, ou pour différentes familles de bactéries, ou pour différentes espèces bactériennes ou pour différentes souches bactériennes sont mémorisées, au moyen desquels, à partir du développement des deux courbes différentielles $\Delta1(t)$ et $\Delta2(t)$, le type de bactérie présent dans l'échantillon biologique ou sa famille ou souche ou espèce à laquelle elle appartient est déduit, étant également présentes, dans lesdits moyens de mémorisation (28), des secondes données pré-mémorisées (D2) de dépendance entre le développement des deux courbes différentielles $\Delta1(t)$ et $\Delta2(t)$ et une variation correspondante $\delta$ des valeurs de turbidité selon le standard McFarland $\delta(t)$ qui définit un seuil de turbidité souhaité qui est obtenu indépendamment du fait que le seuil de turbidité McFarland souhaité soit atteint à partir de la turbidité absolue, lesdites secondes données de dépendance (D2) étant définies et divisées pour chaque famille ou souche ou espèce à laquelle les bactéries appartiennent, ladite dépendance étant exprimée par la relation $\delta = a1^* \Delta^2 + b1^* \Delta + c1$, où a1, b1 et c1 sont des paramètres spécifiques appropriés de la famille identifiée, lesdits moyens de traitement (26) étant aptes à mettre en corrélation les deux courbes différentielles $\Delta1(t)$ et $\Delta2(t)$ avec la variation correspondante $\delta$ desdites valeurs de turbidité selon le standard McFarland.

Fig. 1

Fig. 2

Fig. 3

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050254055 A